# EUROPEAN PATENT SPECIFICATION

(11) **EP 4 605 380 B1**
(45) Date of publication and mention of the grant of the patent: **25.11.2026**
(21) Application number: 23789563.6
(22) Date of filing: 10.10.2023
(51) Int. Cl.: C07D 251/60, C07C 273/12, C07D 251/62

(54) **MELAMINE PROCESS WITH PURIFICATION OF MELAMINE OFFGAS**
MELAMINVERFAHREN MIT REINIGUNG VON MELAMINABGAS
PROCÉDÉ DE PRODUCTION DE MÉLAMINE AVEC PURIFICATION DE GAZ DE DÉGAGEMENT DE MÉLAMINE

(30) Priority: 21.10.2022 EP 22203109; 23.02.2023 EP 23158351
(43) Date of publication of application: 27.08.2025
(73) Proprietor: CASALE SA, 6900 Lugano (CH)
(72) Inventor: GAMBA, Simone, 24040 Pagazzano (BG) (IT)
(74) Representative: M. Zardi & Co S.A.
(86) International application number: PCT/EP2023/078058
(87) International publication number: WO 2024/083571

(56) References cited:
- EP-A1- 2 907 567
- EP-A1- 3 053 915
- EP-A1- 3 208 264
- US-A1- 2004 073 027
- US-B2- 7 311 759

## Description

### Field of application

The invention is in the field of industrial production of melamine. The invention pertains to a high-pressure melamine synthesis process wherein melamine offgas is washed with urea melt.

### Prior art

Melamine is produced at an industrial scale starting from urea following either a non-catalytic high-pressure (HP) process or a low-pressure (LP) catalytic process. The non-catalytic high-pressure process is considered the most advantageous and is becoming predominant.

In the high-pressure process, a urea melt is reacted at pressure which is generally above 70 bar, typically 75 to 200 bar. The temperature of reaction is typically around 375 °C.

The melamine synthesis section may include two reactors arranged in series wherein the first reactor produces a raw melamine melt and the second reactor removes carbon dioxide from the raw melamine melt using a stripping agent such as ammonia. Said second reactor may be termed post-reactor or stripping reactor. The first reactor and the second reactor may be separate vessels or combined in a single apparatus.

The melamine-containing product stream is sent to further treatments, typically in a plant section operating at a lower pressure than the pressure of the melamine synthesis section. Said treatments may include quenching, purification, crystallization, solid-liquid separation and drying, so that said product stream is converted into a solid melamine product of a desired purity. Typically, melamine purification and crystallization are carried out in an alkaline environment and ammonia or sodium hydroxide are the most commonly used alkaline agents.

Irrespective of the configuration of the synthesis section and of the section of the plant treating the raw melamine melt from the synthesis section itself, the reaction of urea to melamine produces a gaseous stream containing predominantly ammonia and carbon dioxide known as offgas, and further containing some melamine carried by the gas as well as other minor components.

Said offgas, which is liberated during the synthesis of melamine, is also termed "melamine offgas". Typically, the melamine offgas is recycled as a feed material for a tied-in urea plant. A combination of production of urea and melamine is attractive because urea is the reagent for the production of melamine and the offgas released during the synthesis of melamine contain ammonia and carbon dioxide which are the starting products for the urea synthesis. However, recycling the melamine offgas to a urea plant requires a proper purification and recover of melamine contained therein.

US 7,311,759 discloses to purify the melamine offgas with a double-stage scrubbing process. The two scrubbing stages are arranged vertically, the second stage being above the first stage. The melamine offgas is supplied to the first stage and a urea melt is introduced from above into the second stage. The urea melt traverses the two stages in countercurrent with the offgas. The contact between the offgas and the falling urea melt generates melamine precursors such as ammeline and cyanuric acid. Accordingly, a urea melt containing ammonia and melamine precursors is withdrawn from the bottom of the scrubber and partially recirculated to the first stage. The recirculated portion of urea melt is cooled in a urea melt cooler prior to reintroduction into the first stage. Cooling the urea melt recovers the heat released by the scrubbing process, particularly by the absorption of the offgas in the urea melt.

The non-recirculated portion of urea melt containing the melamine precursors is sent to the melamine synthesis section. This provides an increase in the energy efficiency of the process because less energy is required for the formation of melamine starting from said precursors rather than from pure urea melt.

Therefore, the above-described double stage scrubbing process is attractive and improves the energy efficiency of the process; however, it still has a few disadvantages.

The melamine contained in the offgas fed to the scrubber can react with said precursors, especially with cyanuric acid, to generate melamine cyanurate. Melamine cyanurate has a low solubility in urea melt under the operating temperature of the scrubber and may precipitate as melamine cyanurate, particularly in the above-mentioned urea melt cooler of the recirculation line. Said precipitation may be detrimental to the operation of the cooler. Accumulation of the melamine cyanurate may obstruct the recirculation flow and reduce the heat exchange coefficient. The decrease of the heat transfer coefficient and/or of the recirculation flow may also increase the temperature of the urea melt in the cooler and, consequently, in the first stage, which increases the risk of corrosion in the heat exchanger or in the offgas scrubber, particularly at bottom of the scrubber. The above drawbacks may render the urea melt cooler unserviceable and cause a complete shutdown of the plant.

Another issue of the above-mentioned purification process is associated with the high investment cost of the scrubber because it requires a scrubber of large size to be able to accommodate the two stages.

EP 2 907 567 discloses a process for the high-pressure synthesis of melamine using a combined reactor; EP 3 208 264 and EP 3 053 915 disclose methods for revamping a high-pressure melamine plant; US 2004/0073027 discloses a method for purifying the offgas of a high-pressure melamine manufacturing installation in a urea wet scrubber.

### Summary of the invention

The invention aims to overcome the above drawbacks of the prior art.

The aim is reached with a process for the synthesis of melamine according to claim 1.

The process of the invention includes subjecting the melamine offgas to a purification process by washing the offgas with urea melt, to obtain a purified offgas and a urea melt which, as a result of the washing process, contains ammonia and melamine precursors. Said purification process is performed at a pressure of 50 to 200 bar.

The purification process of the invention is performed in a single purification stage, said offgas to be purified and said urea melt being introduced in said single purification stage, and said purified offgas being extracted from said single purification stage.

The urea melt used for the offgas washing process in the purification stage includes a fresh urea melt and a recirculated urea melt, wherein said recirculated urea melt is withdrawn from said purification stage after contact with the offgas, subjected to a cooling process and reintroduced in said purification stage after said cooling process. Said cooling process is performed in a shell and tube heat exchanger. In the cooling process, said recirculated urea melt, optionally added with said fresh urea melt, is cooled to a temperature equal to or above (that is, not less than) a minimum temperature of 165 °C. In certain embodiments said minimum temperature may be 170 °C or 175 °C or 180 °C. The temperature to which the recirculated urea melt is cooled is in the range 165 °C to 245 °C, preferably in the range 170 °C to 235 °C. Other preferred ranges for said temperature are 175 °C to 225 °C or 180 °C to 220 °C. Preferably said temperature is the temperature of the urea melt measured at the outlet of the heat exchanger.

In a preferred embodiment, the mass ratio between said recirculated urea melt and the solid melamine product of the melamine plant, wherein the single-stage purification of the offgas is operated, is between 11 and 30. In preferred embodiments of the invention, said mass ratio may be 13 to 28 or 14 to 22. Said solid melamine product is the solid melamine obtained after the treatment of the raw melamine melt at a pressure lower than the synthesis pressure, for example after quenching, purification, crystallization, separation of the so obtained crystals of melamine from the remaining liquor and drying of said crystals. The mass rate of said solid melamine product normally denotes the capacity of the plant.

The selected temperature in the range 165 °C to 245 °C, to which the recirculated urea melt is cooled prior to reintroduction in said single stage, prevents the precipitation of melamine cyanurate and corrosion issues. Particularly, keeping the recirculated urea melt above 165 °C avoids the precipitation of the cyanurate, whereas a temperature not greater than 245 °C is appropriate to avoid corrosion, i.e. to control the corrosive effect of the urea melt flowing through the urea melt cooler and added to the purification stage. The applicant has found that the bottom of the offgas scrubber is particularly exposed to the risk of corrosion and, thanks to the present invention, this risk is reduced. Accordingly, the risk of unwanted shutdowns of the plant is reduced, the efficiency of the purification process can be maintained in the optimal range.

A noticeable feature of the present invention is performing the offgas washing in a single stage instead of two stages in series, in combination with the above cooling process. The applicant has found that cooling the recirculated urea melt at a temperature in the above-mentioned range can prevent or at least reduce the precipitation of solid melamine cyanurate. Additionally, the applicant has found that a single purification stage can have a washing efficiency comparable to that of a two-stage, in spite of the considerably less cost and complication.

In the present invention, the single stage of purification, which is the only stage of purification of the melamine offgas, receives the melamine offgas to be purified, the recirculated urea melt and the fresh urea melt. The purified offgas is also withdrawn from the same stage. The invention has no arrangement of two purification stages, such as a first stage and second stage located one over the other. In the invention, the recirculated urea melt and the fresh urea melt are not introduced in separate stages of purification, they are introduced instead in the same and only stage of purification.

The above-mentioned mass ratio between the recirculated urea melt and the amount of solid melamine produced (melamine plant capacity) provides optimal contact between urea and melamine offgas relative to size of the apparatus and power required for pumping.

A further aspect of the invention is a combined process for production of urea and melamine according to the claims.

### Description of the invention

The invention concerns a process for the synthesis of melamine. A feed stream of urea melt is reacted under non-catalytic high pressure melamine synthesis conditions to generate a raw melamine product and a stream of melamine offgas comprising ammonia, carbon dioxide, melamine and minor components. The synthesis pressure is preferably 70 bar or above, for example 70 bar to 200 bar.

Said melamine offgas is subjected to a purification process, typically to allow recycling the offgas to a tied-in urea plant. The purification process is basically a washing process (also termed scrubbing process) with a urea melt. The offgas purification process is performed at a high pressure, preferably of at least 50 bar and more preferably equal to the melamine synthesis pressure.

The purification process is performed in a single stage wherein the offgas is washed with urea melt, to obtain a purified offgas and a urea melt containing ammonia and melamine precursors.

The process further comprises to withdraw a stream of urea melt from said single purification stage after contact with the offgas; said stream of urea melt, which contains ammonia and melamine precursors, is cooled and reintroduced in the purification stage after cooling.

The recirculated urea melt is cooled in a shell and tube heat exchanger to a temperature in the range 165 °C to 245 °C, and preferably 170 °C to 235 °C.

In some embodiments the recirculated urea melt is cooled in the above-mentioned heat exchanger to a temperature of not less than 170 °C or not less than 175 °C or not less than 180 °C. Preferably said temperature is also not greater than 235 °C or 225 °C or 220 °C. Said temperature is preferably 170 to 235 °C or 175 to 225 °C or 180 to 220 °C. The lower limits and upper limits of the above-mentioned ranges may be combined. For example, further embodiments include that said temperature is 170 to 245 °C or 175 to 245 °C or 175 to 235 °C or 180 to 245 °C or 180 to 235 °C.

Depending on the temperature to which the recirculated urea melt is cooled, the temperature of the steam produced in the heat exchanger may be 170 to 220 °C or 175 to 220 °C or 170 to 215 °C or 175 to 215 °C.

Preferably, the recirculated urea melt is mixed with a stream of fresh urea melt and the so obtained stream of fresh urea melt and recirculated urea melt is reintroduced in the purification stage. The mixing of the recirculated urea melt and fresh urea melt can be made before or after cooling. Accordingly, the recirculated urea melt can be mixed with the fresh urea melt prior to enter the shell and tube heat exchanger or downstream of said heat exchanger.

The fresh urea melt denotes for example the urea melt obtainable from a urea plant after recovery of unreacted matter and evaporation of water. Typically, the urea melt contains at least 96% urea, the balance being residual water and unavoidable impurities. In contrast, the urea melt withdrawn from the purification stage after contact with the offgas contains a significant amount of ammonia and melamine precursors.

Preferably the shell-and-tube heat exchanger, used to cool the recirculated urea melt, is arranged in a recirculation line that is external to said single purification stage and the fresh urea melt is added to said recirculated urea melt at an injection point which outside the purification stage, either upstream or downstream of said shell and tube heat exchanger.

The fresh urea melt and the recirculated urea melt can be introduced in the single stage of purification separately or together as a mixed stream. If introduced separately, the fresh urea melt and the recirculated urea melt enter the purification stage with separate lines feeding respective separate sprayers.

According to an embodiment, in the purification stage the urea melt and the recirculated urea melt are sprayed, separately or together in a mixed stream, from top of the purification stage and the offgas to be purified is introduced in a lower section of said purification stage, so that the offgas flows upward in countercurrent with the falling urea melt.

According to an interesting embodiment, the purification step is carried out in the temperature range 170-240 °C, preferably 175-240 °C. Particularly, in a preferred embodiment, the urea melt withdrawn from bottom of the purification stage has a temperature in the range 170-250 °C or 175-240 °C.

Preferably the stream of recirculated urea melt, possibly mixed with fresh urea melt, is cooled in the tube side of the heat exchanger. The heat removed from the urea melt in the heat exchanger can be used to produce steam in the shell side of said heat exchanger. The temperature of said steam produced in said shell side of the heat exchanger is preferably between 160 °C to 240 °C, more preferably 165 °C to 230 °C, and preferably said steam is saturated steam at a pressure of least 6 barg.

Said temperature of the steam production in the shell side is selected to keep the inner surface of the tubes of the heat exchanger above the temperature at which precipitation of melamine cyanurate starts. The applicant has found that the inner surface of tubes, which is contact with the recirculated urea melt, is the point most exposed to the unwanted precipitation of melamine cyanurate. Furthermore, a melamine plant typically comprises a steam network at around 6 barg, which corresponds to 165 °C, so that the production of steam in the urea melt cooler seamlessly integrates with the steam network of the melamine plant.

The applicant has found that cooling the urea melt to not less than 165 °C is advantageous contrary to the customary practice which prompts to cool said recirculated stream as much as possible insofar the urea is above its melting temperature, to recover heat and reduce the recirculated flow rate. The applicant has judiciously found that keeping the recirculated urea melt above 165 °C reduces the drawbacks connected to precipitation of melamine cyanurate in the urea melt cooler so that the apparent disadvantage in terms of increased recirculated flowrate is well compensated.

The temperature of the shell-side steam is also in contrast to the customary approach of heat exchanger design, which would prompt to produce steam at a temperature and pressure as low as possible, to increase the difference of temperature across the urea melt cooler and make said cooler smaller.

The non-recirculated portion of the urea melt collected from the purification stage may be added with fresh urea melt to form the feed of the melamine synthesis section. According to an embodiment, the urea melt feed stream supplied to the high-pressure melamine synthesis section includes an amount of the urea melt which is supplied to the purification stage and an amount of the urea melt containing ammonia and melamine precursors which is withdrawn from the purification stage. A portion of the urea melt containing ammonia and melamine precursors withdrawn from the purification stage is recycled to the purification stage and another portion is conveyed to the high-pressure melamine synthesis section.

The purification process may include addition of carbon dioxide. In an embodiment, a carbon dioxide stream is added to the melamine offgas which is conveyed to the purification stage. In another embodiment a carbon dioxide stream is introduced directly into the purification stage.

According to a preferred embodiment, the synthesis of melamine includes a conversion step and a stripping step, wherein said conversion step includes reacting said urea melt feed stream under suitable melamine synthesis conditions to generate a raw melamine product and said stripping step includes the stripping of said raw melamine product in the presence of gaseous ammonia, to remove carbon dioxide contained in the raw melamine.

The conversion of urea into melamine is performed in a melamine synthesis section. In some embodiments, said melamine synthesis section includes a single reactor, from which the raw melamine and the melamine offgas are withdrawn. In other embodiments, said melamine synthesis section includes a primary reactor where urea melt is reacted, followed by a secondary reactor where the melamine-containing effluent of the primary reactor is stripped with gaseous ammonia. In such embodiments, each of the primary reactor and the secondary reactor produce a respective stream of melamine offgas. Both streams of melamine offgas are made predominantly of ammonia and carbon dioxide, although they may differ in composition.

The melamine offgas subject to scrubbing with urea melt, in accordance with embodiments the invention, may include only the melamine offgas stream from the primary reactor or both melamine offgas streams from the primary reactor and secondary reactor, possibly combined into a single stream. In a further embodiment, a combined reactor performs the function of the primary reactor and secondary reactor; to this purpose, said combined reactor includes a primary reaction stage and a secondary reaction stage.

According to an embodiment, the off-gas is introduced via an off-gas distributor above or below a liquid level of the urea melt containing ammonia and melamine precursors. A preferred embodiment of said offgas distributor is disclosed in US 7,311,759.

According to another embodiment, the purification process is carried out in a scrubber and said urea melt, prior to be injected into the purification stage, is divided into a plurality of sub-streams and introduced at multiple locations e.g. at multiple heights in the purification stage of said scrubber.

In a combined urea-melamine embodiment, ammonia and carbon dioxide are reacted to form a urea solution in a urea synthesis section, the urea solution is processed in at least one recovery section to obtain a purified urea solution and water is removed from the solution to form a urea melt. Said urea melt is used in the above-described process for synthesis of melamine. The melamine offgas generated during the synthesis of melamine is recycled to the production of urea.

The melamine synthesis is performed at a synthesis pressure which is typically above 70 bar, such as 70 to 200 bar and preferably 75 to 200 bar. The purification of the melamine offgas is performed at a pressure in the range 50 to 200 bar. Preferably, the purification of the melamine offgas is performed substantially at synthesis pressure. The offgas purification process may be performed at a pressure slightly less than the melamine synthesis pressure, wherein the difference is not more than 20 bar or not more than 5 bar. Accordingly, also the recirculated urea melt is typically at a pressure of 50 to 200 bar. Pressure is given in bar gauge.

### Examples

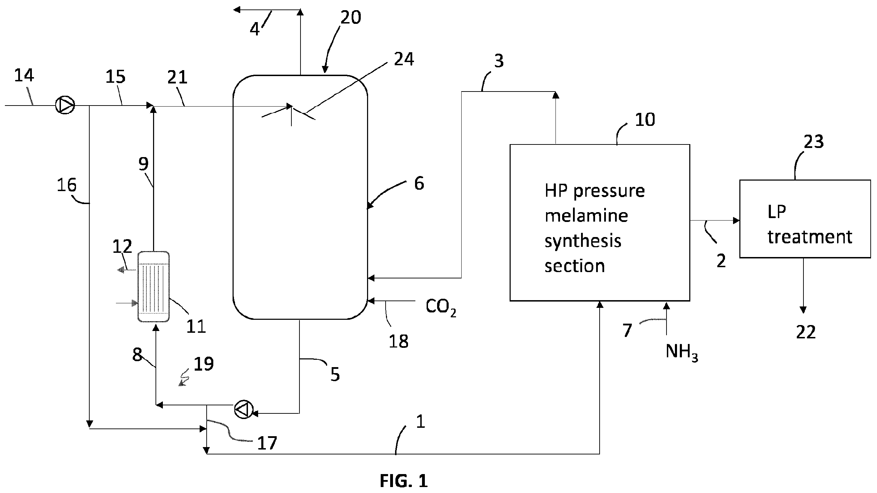

Table 1 summarizes several experimental tests with a different temperature of the recirculated urea melt after cooling, measured at the outlet of the heat exchanger. For each test the expected lifetime of the heat exchanger and the scrubber (bottom part) was estimated and the occurrence of melamine cyanurate precipitation in the heat exchanger was also identified. The table refers to an embodiment where the fresh urea melt mixes with the cooled recirculated urea melt, such as in Fig. 1.

In the experimental tests of cases 1 to 3 and 5 to 7, the temperature of the recirculated urea melt was within the range 175 °C to 224 °C, no precipitation of melamine cyanurate occurred in the heat exchanger and an expected lifetime of the heat exchanger greater than 15 years was estimated. In the comparative test of case 4, the temperature of the recirculated urea melt at the outlet of the heat exchanger was 156 °C and fouling of the heat exchanger was clearly detected. The shell side steam temperature was higher than 160 °C in the cases 1 to 3 and 5 to 7 and lower than 160°C in the comparative case 4.

**Table 1**

| | Case 1 | | Case 2 | | Case 3 | | Case 4 (comparative) | |
|---|---|---|---|---|---|---|---|---|
| Temperature [°C] | Offgas | Urea | Offgas | Urea | Offgas | Urea | Offgas | Urea |
| Inlet | 370 | 150 | 370 | 150 | 370 | 150 | 370 | 150 |
| Exit | 225 | 225 | 242 | 242 | 225 | 225 | 225 | 225 |
| Specific heat for steam production [kJ/mol urea] | 26 | | 21 | | 26 | | 26 | |
| Content of melamine precursors and condensed melamine in the urea melt fed to the melamine reactor | 5.5% | | 6.5% | | 5.5% | | 5.5% | |
| Fresh urea inlet [t_{UREA}/t_{MELAMINE}] | 3.1 | | 3.1 | | 3.1 | | 3.1 | |
| Circulating urea [t_{UREA}/t_{MELAMINE}] | 20.5 | | 20.5 | | 32.0 | | 7.0 | |
| Temperature recirculated cooled urea melt [°C] | 202 | | 224 | | 210 | | 156 | |
| Melamine removal from the offgas (efficiency) | 100% | | 100% | | 100% | | 90% | |
| Pumping energy ratio | | 1.0 | | 1.0 | | 1.6 | | - |
| Melamine cyanurate precipitation in the heat exchanger | | No | | No | | No | | Yes |
| Expected equipment lifetime (years) | | >=20 | | 15-20 | | >=20 | | - |

**Table 1 (continues)**

| | | Case 5 | | Case 6 | | Case 7 | |
|---|---|---|---|---|---|---|---|
| Temperature [°C] | | Offgas | Urea | Offgas | Urea | Offgas | Urea |
| | Inlet | 370 | 150 | 370 | 150 | 370 | 150 |
| | Outlet | 205 | 205 | 225 | 225 | 205 | 205 |
| Specific heat for steam production [kJ/mol urea] | | 33 | | 26 | | 33 | |
| Content of melamine precursors and condensed melamine in the urea melt fed to the melamine reactor | | 4% | | 5.5% | | 4.0% | |
| Fresh urea inlet [t_{UREA}/t_{MELAMINE}] | | 3.1 | | 3.1 | | 3.1 | |
| Circulating urea in the first stage [t_{UREA}/t_{MELAMINE}] | | 20.5 | | 14.0 | | 28.0 | |
| Temperature of recirculated cooled urea melt [°C] | | 175 | | 191 | | 183 | |
| Melamine removal from the offgas (efficiency) | | 100% | | 100% | | 100% | |
| Pumping energy ratio | | 1 | | 0.7 | | 1.4 | |
| Melamine cyanurate precipitation in the heat exchanger | | No | | No | | No | |
| Expected equipment lifetime (years) | | >=20 | | >=20 | | >=20 | |

### Description of the figures

Fig. 1 is a schematic representation of a melamine synthesis process according to an embodiment of the invention wherein the fresh urea melt and the recirculated urea melt are mixed after cooling.
Fig. 2 illustrates an embodiment wherein the fresh urea melt and the recirculated urea melt are mixed before cooling.

Fig. 1 illustrates a high-pressure melamine synthesis section 10 and a melamine offgas purification section comprising a scrubber 20.

The high-pressure melamine synthesis section 10 is supplied with a urea melt feed stream 1 and gaseous ammonia 7.

In the melamine synthesis section 10 the urea feed stream 1 is reacted under high-pressure synthesis conditions to generate a raw melamine product 2 and melamine offgas 3. Said melamine offgas 3 contains carbon dioxide, ammonia, some residual melamine and other minor components. Ammonia 7 is injected in the synthesis section 10 to act as a stripping agent to remove carbon dioxide from the raw melamine.

The synthesis section 10 may comprise two separate reactors wherein in the first reactor raw melamine is synthesized and in the second reactor ammonia is used as a stripping agent to remove the carbon dioxide from the raw melamine. Alternatively, the synthesis of raw melamine and stripping with ammonia can be carried out in a single reactor. In a preferred embodiment, a single reactor has coaxial zones for synthesis and stripping. For example, the synthesis of melamine is carried out in a central zone of the reactor and stripping is carried out in an annular zone wrapped around said central zone.

The melamine offgas 3 is sent to a scrubber 20. Said scrubber 20 comprises a single purification stage 6. The purification stage 6 receives, from bottom to top, a stream of gaseous carbon dioxide 18, the melamine offgas 3 and a washing urea melt 21. Said washing urea melt 21 includes a fresh urea melt 15 and a recirculated urea melt 9, which is taken from bottom of the scrubber 20 and cooled in a heat exchanger 11. Said washing urea melt 21 is distributed with a sprayer 24 from top of the purification stage 6.

The fresh urea melt 15 is a portion of a urea melt 14 coming from a tied-in urea plant (which is not shown in the figure).

The scrubber 20 is traversed in counter-current by the upward flowing offgas and by the sprayed urea melt.

Effluents of the scrubber 20 are a purified offgas 4 and a urea melt 5 containing ammonia and melamine precursors. The purified offgas 4 is withdrawn from top of the purification stage 6, whereas said urea melt 5 containing ammonia and melamine precursors is collected from bottom of the purification stage 6.

Once extracted, said urea melt 5 is separated into a first portion 8 and a second portion 17. The first portion 8 is recycled to the purification stage 6 after cooling in a shell-and-tube heat exchanger 11. Particularly the urea melt is recirculated via a recirculation line 19 including said heat exchanger 11. The cooled urea melt 9 leaves the heat exchanger 11 at a temperature in the range 165 °C to 245 °C.

The urea melt 8 traverses the tube side of the heat exchanger 11. The shell side of the heat exchanger 11 produces steam 12 with heat removed from the urea melt 8. Preferably the temperature of said steam 12 is between 160 °C and 240 °C. Particularly preferably, said steam 12 is saturated steam at least 6 barg.

The scrubber 20 works as follows: the ascending stream of melamine offgas 3 is washed and purified by counter-current contact with the urea melt stream 21, which contains the recirculated and cooled urea melt 9 loaded with ammonia and melamine precursors as well as the fresh urea melt 15.

The injection of carbon dioxide 18 promotes the formation of the melamine precursors contained in the urea melt 5. The purified offgas 4 emerging from the top of the purification stage 6 can be recycled to a urea plant not shown in the figure, for example to the urea plant which produces the urea melt 14.

The second portion 17 of the urea melt 5, that is the portion which is not recirculated to the purification stage 6, is mixed with an amount 16 of the urea melt 14 to form the urea melt feed stream 1.

The raw melamine melt 2 is processed in a low-pressure section 23 to obtain solid melamine 22 of a desired purity. Said section 23 preferably includes quenching, purification, crystallization, solid-liquid separation and drying.

In the embodiment of Fig. 1, the fresh urea melt 15 mixes with the recirculated urea melt 9 after cooling. The mixing point is in the line 19 the downstream the heat exchanger 11.

In the embodiment of Fig. 1, the fresh urea melt slightly cools the recirculated stream 9. A precipitation of melamine cyanurate in this point, if any, is generally not problematic because the piping between the heat exchanger 11 and the scrubber 20 has a relatively large diameter being less prone to clogging. Particularly, the diameter is much larger than the diameter of the tubes of the heat exchanger. Fouling is no longer an issue in the piping between the heat exchanger 11 and the scrubber 20 since no heat exchange occurs at this point.

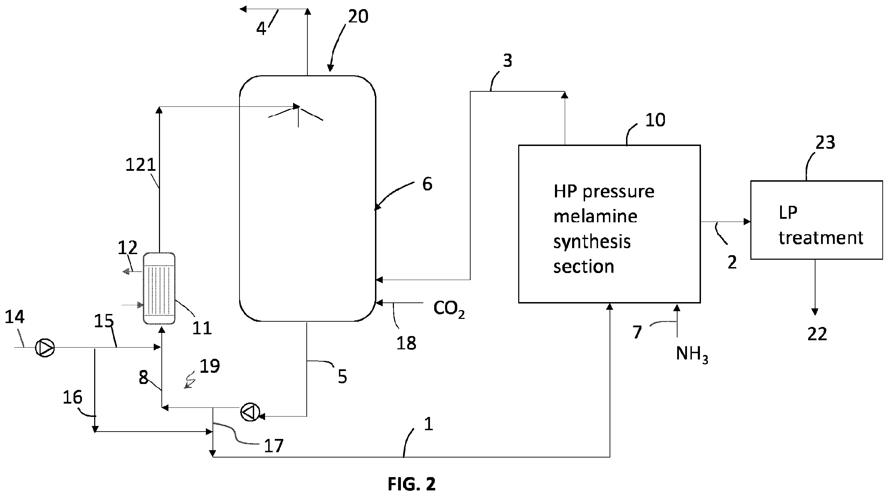

Fig. 2 illustrates an embodiment similar to Fig. 1 but wherein the fresh urea melt 15 mixes with the recirculated urea melt 8 before cooling. Accordingly, the fresh urea melt is added to the recirculated urea melt upstream the heat exchanger 11. The so obtained mixed stream is cooled in the heat exchanger 11 to a temperature in the range 165 °C to 245 °C. The cooled stream 121 exiting the heat exchanger 11 is sent to the purification stage 6.

## Claims

1. A process for the synthesis of melamine including the steps of:
reacting a feed stream (1) of urea melt in a melamine synthesis section (10) under non-catalytic high pressure melamine synthesis conditions to generate a raw melamine product (2) and an offgas (3) comprising ammonia and carbon dioxide,
subjecting said offgas (3) to a purification process by washing the offgas (3) with urea melt (21, 121), to obtain a purified offgas (4) and a urea melt (5) which, as a result of the washing process, contains ammonia and melamine precursors; wherein said purification process is performed at a pressure of 50 to 200 bar;
wherein the purification process is performed in a single purification stage (6), said offgas (3) to be purified and said urea melt (21, 121) being introduced in said single purification stage (6) and said purified offgas (4) being extracted from said single purification stage (6),
wherein the urea melt (21, 121) used for the offgas washing process in the purification stage includes a fresh urea melt (15) and a recirculated urea melt (8),
wherein said recirculated urea melt (8) is withdrawn from said purification stage (6) after contact with the offgas (3), subjected to a cooling process and reintroduced in said purification stage (6) after said cooling process;
wherein said cooling process is performed in a shell and tube heat exchanger (11) and where, in the cooling process, said recirculated urea melt (8), optionally added with said fresh urea melt (15), is cooled to a temperature range of 165 °C to 245 °C.

2. Process according to claim 1, wherein said temperature, to which the recirculated urea melt is cooled, is not less than 170 °C or not less than 175 °C or not less than 180 °C, and preferably not greater than 235 °C or not greater than 225 °C or not greater than 220 °C.

3. Process according to claim 1 or 2, wherein said temperature, to which the recirculated urea melt is cooled, is in any one of the following ranges: 170 °C to 235 °C or 175 °C to 225 °C or 180 °C to 220 °C.

4. Process according to any of claims 1 to 3, wherein said fresh urea melt (15) is mixed with the recirculated urea melt (8) before said cooling process, or said fresh urea melt (15) is mixed with the recirculated urea melt (9) after cooling.

5. Process according to any of claims 1 to 4, wherein said shell and tube heat exchanger (11) is arranged in a recirculation line (19) external to said purification stage (6) and said fresh urea melt (15) is added to said recirculated urea melt (8, 9) at an injection point which is located on the recirculation line (19) upstream or downstream said shell and tube heat exchanger (11).

6. Process according to any of the previous claims, wherein in said purification stage (6) the fresh urea melt (15) and the recirculated urea melt (8, 9), separately or mixed together in a single stream (21, 121), are sprayed from top of the purification stage (6), and the offgas (3) to be purified is introduced in a lower section of said purification stage, so that the offgas (3) flows upward in counter-current with said urea melt (15) and said recirculated urea melt (8).

7. Process according to any of the previous claims, wherein the recirculated urea melt (8) directed to the heat exchanger (11), possibly added with fresh urea melt (15), is cooled in the tube side of said heat exchanger (11) and heat removed from the urea melt in the heat exchanger (11) is used to produce steam (12) in the shell side of said heat exchanger (11).

8. Process according to claim 7, wherein the temperature of said steam (12) produced in said shell side of the heat exchanger (11) is between 160 °C to 240 °C, preferably 165 °C to 230 °C, and preferably said steam (12) is saturated steam at a pressure of at least 6 barg.

9. Process according to any of the previous claims, wherein the urea melt withdrawn from bottom of the purification stage has a temperature in the range 170 °C to 250 °C, preferably 175 °C to 240 °C.

10. Process according to any of the previous claims, wherein said raw melamine melt (2) is processed further to obtain a solid melamine product (22) and wherein the mass ratio between said recirculated urea melt (8, 9) and said solid melamine product (22) is between 11 and 30 or between 13 and 28 or between 14 and 22.

11. Process according to any of the previous claims, wherein said urea melt feed stream (1), which is directed to the melamine synthesis section (10), includes fresh urea melt (16) and a portion (17) of the urea melt (5) containing ammonia and melamine precursors withdrawn from the purification stage (6).

12. Process according to any of the previous claims, wherein carbon dioxide (18) is added to said offgas (3) in said purification stage (6).

13. Process according to any of the previous claims, wherein the synthesis of melamine includes a conversion step and a stripping step, wherein in said conversion step said urea melt feed stream (1) is reacted under high-pressure melamine synthesis conditions to generate a raw melamine product containing carbon dioxide and in said stripping step said raw melamine product containing carbon dioxide is stripped in presence of gaseous ammonia (7).

14. Process according to claim 13, wherein the synthesis of melamine is performed in a synthesis section (10) including a primary reactor followed by a secondary reactor, wherein said stripping step is performed in the secondary reactor, and the offgas (3) subject to said purification process includes offgas withdrawn from the primary reactor only or the offgas subject to said purification process includes offgas withdrawn from the primary reactor and offgas withdrawn from the secondary reactor, or wherein the synthesis of melamine is performed in a synthesis section including a single reactor, and the offgas subjected to said purification process is withdrawn from said single reactor.

15. Process according to any of the previous claims, wherein the offgas (3) is introduced via an offgas distributor above or below a liquid level of urea melt collected at the bottom of the purification stage.

16. A combined process for the synthesis of urea and melamine wherein:
ammonia and carbon dioxide are reacted to form a urea solution in a urea synthesis section; said solution is processed in at least one recovery section to obtain a purified urea solution; water is removed from said solution to form a urea melt (14);
said urea melt (14) is used in a process for synthesis of melamine according to any of claims 1 to 15;
melamine offgas (3) generated during the synthesis of melamine are recycled to the production of urea.

## Patentansprüche

1. Verfahren zur Synthese von Melamin, umfassend die Schritte:
Umsetzen eines Einsatzstroms (1) aus Harnstoffschmelze in einem Melaminsyntheseabschnitt (10) unter nichtkatalytischen Hochdruck-Melaminsynthesebedingungen, um ein rohes Melaminprodukt (2) und ein Abgas (3), umfassend Ammoniak und Kohlendioxid, zu erzeugen,
Unterziehen des Abgases (3) einem Reinigungsprozess durch Waschen des Abgases (3) mit Harnstoffschmelze (21, 121), um ein gereinigtes Abgas (4) und eine Harnstoffschmelze (5) zu erhalten, die infolge des Waschprozesses Ammoniak und Melaminvorläufer enthält; wobei der Reinigungsprozess bei einem Druck von 50 bis 200 bar durchgeführt wird;
wobei der Reinigungsprozess in einer einzigen Reinigungsstufe (6) durchgeführt wird, wobei das zu reinigende Abgas (3) und die Harnstoffschmelze (21, 121) in die einzige Reinigungsstufe (6) eingebracht werden und das gereinigte Abgas (4) aus der einzigen Reinigungsstufe (6) entnommen wird,
wobei die Harnstoffschmelze (21, 121), die für den Abgaswaschprozess in der Reinigungsstufe verwendet wird, eine frische Harnstoffschmelze (15) und eine rezirkulierte Harnstoffschmelze (8) umfasst,
wobei die rezirkulierte Harnstoffschmelze (8) nach Kontakt mit dem Abgas (3) aus der Reinigungsstufe (6) abgezogen, einem Kühlprozess unterzogen und nach dem Kühlprozess wieder in die Reinigungsstufe (6) eingebracht wird;
wobei der Kühlprozess in einem Rohrbündelwärmetauscher (11) durchgeführt wird und wobei in dem Kühlprozess die rezirkulierte Harnstoffschmelze (8), der gegebenenfalls die frische Harnstoffschmelze (15) zugesetzt ist, auf einen Temperaturbereich von 165 °C bis 245 °C gekühlt wird.

2. Verfahren nach Anspruch 1, wobei die Temperatur, auf welche die rezirkulierte Harnstoffschmelze gekühlt wird, nicht weniger als 170 °C oder nicht weniger als 175 °C oder nicht weniger als 180 °C beträgt und vorzugsweise nicht mehr als 235 °C oder nicht mehr als 225 °C oder nicht mehr als 220 °C beträgt.

3. Verfahren nach Anspruch 1 oder 2, wobei die Temperatur, auf welche die rezirkulierte Harnstoffschmelze gekühlt wird, in einem der folgenden Bereiche liegt: 170 °C bis 235 °C oder 175 °C bis 225 °C oder 180 °C bis 220 °C.

4. Verfahren nach einem der Ansprüche 1 bis 3, wobei die frische Harnstoffschmelze (15) vor dem Kühlprozess mit der rezirkulierten Harnstoffschmelze (8) gemischt wird oder die frische Harnstoffschmelze (15) nach dem Kühlen mit der rezirkulierten Harnstoffschmelze (9) gemischt wird.

5. Verfahren nach einem der Ansprüche 1 bis 4, wobei der Rohrbündelwärmetauscher (11) in einer Rezirkulationsleitung (19) außerhalb der Reinigungsstufe (6) angeordnet ist und die frische Harnstoffschmelze (15) der rezirkulierten Harnstoffschmelze (8, 9) an einer Einspritzstelle zugesetzt wird, die an der Rezirkulationsleitung (19) stromaufwärts oder stromabwärts des Rohrbündelwärmetauschers (11) angeordnet ist.

6. Verfahren nach einem der vorhergehenden Ansprüche, wobei in der Reinigungsstufe (6) die frische Harnstoffschmelze (15) und die rezirkulierte Harnstoffschmelze (8, 9), getrennt oder miteinander in einem einzigen Strom (21, 121) gemischt, von oben in die Reinigungsstufe (6) eingesprüht werden und das zu reinigende Abgas (3) in einen unteren Abschnitt der Reinigungsstufe eingebracht wird, so dass das Abgas (3) im Gegenstrom zu der Harnstoffschmelze (15) und der rezirkulierten Harnstoffschmelze (8) nach oben strömt.

7. Verfahren nach einem der vorhergehenden Ansprüche, wobei die dem Wärmetauscher (11) zugeführte rezirkulierte Harnstoffschmelze (8), der gegebenenfalls frische Harnstoffschmelze (15) zugesetzt ist, auf der Rohrseite des Wärmetauschers (11) gekühlt wird und der der Harnstoffschmelze in dem Wärmetauscher (11) entzogene Wärmeanteil dazu verwendet wird, auf der Mantelseite des Wärmetauschers (11) Dampf (12) zu erzeugen.

8. Verfahren nach Anspruch 7, wobei die Temperatur des auf der Mantelseite des Wärmetauschers (11) erzeugten Dampfes (12) zwischen 160 °C und 240 °C, vorzugsweise 165 °C bis 230 °C, liegt und wobei der Dampf (12) vorzugsweise Sattdampf bei einem Druck von mindestens 6 barg ist.

9. Verfahren nach einem der vorhergehenden Ansprüche, wobei die vom Boden der Reinigungsstufe abgezogene Harnstoffschmelze eine Temperatur im Bereich von 170 °C bis 250 °C, vorzugsweise 175 °C bis 240 °C, aufweist.

10. Verfahren nach einem der vorhergehenden Ansprüche, wobei die rohe Melaminschmelze (2) weiterverarbeitet wird, um ein festes Melaminprodukt (22) zu erhalten, und wobei das Massenverhältnis zwischen der rezirkulierten Harnstoffschmelze (8, 9) und dem festen Melaminprodukt (22) zwischen 11 und 30 oder zwischen 13 und 28 oder zwischen 14 und 22 liegt.

11. Verfahren nach einem der vorhergehenden Ansprüche, wobei der Harnstoffschmelze-Einsatzstrom (1), der dem Melaminsyntheseabschnitt (10) zugeführt wird, frische Harnstoffschmelze (16) und einen Anteil (17) der aus der Reinigungsstufe (6) abgezogenen Harnstoffschmelze (5), die Ammoniak und Melaminvorläufer enthält, umfasst.

12. Verfahren nach einem der vorhergehenden Ansprüche, wobei Kohlendioxid (18) dem Abgas (3) in der Reinigungsstufe (6) zugesetzt wird.

13. Verfahren nach einem der vorhergehenden Ansprüche, wobei die Synthese von Melamin einen Umwandlungsschritt und einen Stripp-Schritt umfasst, wobei in dem Umwandlungsschritt der Harnstoffschmelze-Einsatzstrom (1) unter Hochdruck-Melaminsynthesebedingungen umgesetzt wird, um ein Kohlendioxid enthaltendes rohes Melaminprodukt zu erzeugen, und in dem Stripp-Schritt das Kohlendioxid enthaltende rohe Melaminprodukt in Gegenwart von gasförmigem Ammoniak (7) gestrippt wird.

14. Verfahren nach Anspruch 13, wobei die Synthese von Melamin in einem Syntheseabschnitt (10) durchgeführt wird, der einen Primärreaktor, gefolgt von einem Sekundärreaktor, umfasst, wobei der Stripp-Schritt in dem Sekundärreaktor durchgeführt wird und das dem Reinigungsprozess unterzogene Abgas (3) nur aus dem Primärreaktor abgezogenes Abgas umfasst oder das dem Reinigungsprozess unterzogene Abgas aus dem Primärreaktor abgezogenes Abgas und aus dem Sekundärreaktor abgezogenes Abgas umfasst, oder wobei die Synthese von Melamin in einem Syntheseabschnitt durchgeführt wird, der einen einzigen Reaktor umfasst, und das dem Reinigungsprozess unterzogene Abgas aus dem einzigen Reaktor abgezogen wird.

15. Verfahren nach einem der vorhergehenden Ansprüche, wobei das Abgas (3) über einen Abgasverteiler oberhalb oder unterhalb eines Flüssigkeitsspiegels von am Boden der Reinigungsstufe gesammelter Harnstoffschmelze eingebracht wird.

16. Kombiniertes Verfahren zur Synthese von Harnstoff und Melamin, wobei:
Ammoniak und Kohlendioxid umgesetzt werden, um in einem Harnstoffsyntheseabschnitt eine Harnstofflösung zu bilden; die Lösung in mindestens einem Rückgewinnungsabschnitt verarbeitet wird, um eine gereinigte Harnstofflösung zu erhalten; Wasser aus der Lösung entfernt wird, um eine Harnstoffschmelze (14) zu bilden;
die Harnstoffschmelze (14) in einem Verfahren zur Synthese von Melamin nach einem der Ansprüche 1 bis 15 verwendet wird;
während der Synthese von Melamin erzeugtes Melaminabgas (3) zur Herstellung von Harnstoff zurückgeführt wird.

## Revendications

1. Procédé pour la synthèse de mélamine incluant les étapes de :
la mise en réaction d'un courant d'apport (1) de masse fondue d'urée dans une section de synthèse de mélamine (10) dans des conditions de synthèse de mélamine non catalytiques à haute pression pour générer un produit de mélamine brut (2) et un gaz résiduel (3) comportant de l'ammoniac et du dioxyde de carbone,
la soumission dudit gaz résiduel (3) à un procédé de purification par le lavage du gaz résiduel (3) avec une masse fondue d'urée (21, 121) pour obtenir un gaz résiduel purifié (4) et une masse fondue d'urée (5) qui, en conséquence du procédé de lavage, contient de l'ammoniac et des précurseurs de mélamine ; dans lequel ledit procédé de purification est réalisé à une pression de 50 à 200 bar ;
dans lequel le procédé de purification est réalisé dans un étage de purification unique (6), ledit gaz résiduel (3) devant être purifié et ladite masse fondue d'urée (21, 121) étant introduits dans ledit étage de purification unique (6) et ledit gaz résiduel purifié (4) étant extrait à partir dudit étage de purification unique (6),
dans lequel la masse fondue d'urée (21, 121) utilisée pour le procédé de lavage de gaz résiduel dans l'étage de purification inclut une masse fondue d'urée neuve (15) et une masse fondue d'urée mise en recirculation (8),
dans lequel ladite masse fondue d'urée mise en recirculation (8) est prélevée à partir dudit étage de purification (6) après contact avec le gaz résiduel (3), soumise à un procédé de refroidissement et réintroduite dans ledit étage de purification (6) après ledit procédé de refroidissement ;
dans lequel ledit procédé de refroidissement est réalisé dans un échangeur de chaleur (11) à calandre et à tubes et où, dans le procédé de refroidissement, ladite masse fondue d'urée mise en recirculation (8), à laquelle est facultativement ajoutée ladite masse fondue d'urée neuve (15), est refroidie jusqu'à une plage de températures de 165 °C à 245 °C.

2. Procédé selon la revendication 1, dans lequel ladite température, jusqu'à laquelle la masse fondue d'urée mise en recirculation est refroidie, n'est pas inférieure à 170 °C ou n'est pas inférieure à 175 °C ou n'est pas inférieure à 180 °C, et de préférence n'est pas supérieure à 235 °C ou n'est pas supérieure à 225 °C ou n'est pas supérieure à 220 °C.

3. Procédé selon la revendication 1 ou 2, dans lequel ladite température, jusqu'à laquelle la masse fondue d'urée mise en recirculation est refroidie, est dans l'une quelconque des plages suivantes : de 170 °C à 235 °C ou de 175 °C à 225 °C ou de 180 °C à 220 °C.

4. Procédé selon l'une quelconque des revendications 1 à 3, dans lequel ladite masse fondue d'urée neuve (15) est mélangée avec la masse fondue d'urée mise en recirculation (8) avant ledit procédé de refroidissement, ou ladite masse fondue d'urée neuve (15) est mélangée avec la masse fondue d'urée mise en recirculation (9) après refroidissement.

5. Procédé selon l'une quelconque des revendications 1 à 4, dans lequel ledit échangeur de chaleur (11) à calandre et à tubes est agencé dans une conduite de mise en recirculation (19) externe audit étage de purification (6) et ladite masse fondue d'urée neuve (15) est ajoutée à ladite masse fondue d'urée mise en recirculation (8, 9) au niveau d'un point d'injection qui est situé sur la conduite de recirculation (19) en amont ou en aval dudit échangeur de chaleur (11) à calandre et à tubes.

6. Procédé selon l'une quelconque des revendications précédentes, dans lequel, dans ledit étage de purification (6), la masse fondue d'urée neuve (15) et la masse fondue d'urée mise en recirculation (8, 9), séparément ou mélangées ensemble en un courant unique (21, 121), sont pulvérisées depuis le haut de l'étage de purification (6), et le gaz résiduel (3) devant être purifié est introduit dans une section inférieure dudit étage de purification, de sorte que le gaz résiduel (3) s'écoule vers le haut à contre-courant par rapport à ladite masse fondue d'urée neuve (15) et ladite masse fondue d'urée mise en recirculation (8).

7. Procédé selon l'une quelconque des revendications précédentes, dans lequel la masse fondue d'urée mise en recirculation (8) dirigée vers l'échangeur de chaleur (11), à laquelle est éventuellement ajoutée la masse fondue d'urée neuve (15), est refroidie dans le côté tubes dudit échangeur de chaleur (11) et de la chaleur retirée de la masse fondue d'urée dans l'échangeur de chaleur (11) est utilisée pour produire de la vapeur (12) dans le côté calandre dudit échangeur de chaleur (11).

8. Procédé selon la revendication 7, dans lequel la température de ladite vapeur (12) produite dans ledit côté calandre de l'échangeur de chaleur (11) est entre 160 °C et 240 °C, de préférence 165 °C et 230 °C, et de préférence ladite vapeur (12) est de la vapeur saturée à une pression d'au moins 6 barg.

9. Procédé selon l'une quelconque des revendications précédentes, dans lequel la masse fondue d'urée prélevée à partir du bas de l'étage de purification possède une température dans la plage de 170 °C à 250 °C, de préférence de 175 °C à 240 °C.

10. Procédé selon l'une quelconque des revendications précédentes, dans lequel ladite masse fondue de mélamine brute (2) est davantage traitée pour obtenir un produit de mélamine solide (22) et dans lequel le rapport massique entre ladite masse fondue d'urée mise en recirculation (8, 9) et ledit produit de mélamine solide (22) est entre 11 et 30 ou entre 13 et 28 ou entre 14 et 22.

11. Procédé selon l'une quelconque des revendications précédentes, dans lequel ledit courant d'apport (1) de masse fondue d'urée, qui est dirigé vers la section de synthèse de mélamine (10), inclut une masse fondue d'urée neuve (16) et une partie (17) de la masse fondue d'urée (5) contenant de l'ammoniac et des précurseurs de mélamine prélevés à partir de l'étage de purification (6).

12. Procédé selon l'une quelconque des revendications précédentes, dans lequel du dioxyde de carbone (18) est ajouté audit gaz résiduel (3) dans ledit étage de purification (6).

13. Procédé selon l'une quelconque des revendications précédentes, dans lequel la synthèse de mélamine inclut une étape de conversion et une étape de réextraction, dans lequel, à ladite étape de conversion, ledit courant d'apport (1) de masse fondue d'urée est mis en réaction dans des conditions de synthèse de mélamine à haute pression pour générer un produit de mélamine brut contenant du dioxyde de carbone et, à ladite étape de réextraction, ledit produit de mélamine brut contenant du dioxyde de carbone est réextrait en présence d'ammoniac gazeux (7).

14. Procédé selon la revendication 13, dans lequel la synthèse de mélamine est réalisée dans une section de synthèse (10) incluant un réacteur primaire suivi d'un réacteur secondaire, dans lequel ladite étape de réextraction est réalisée dans le réacteur secondaire, et le gaz résiduel (3) soumis audit procédé de purification inclut un gaz résiduel prélevé à partir du réacteur primaire uniquement ou le gaz résiduel soumis audit procédé de purification inclut un gaz résiduel prélevé à partir du réacteur primaire et un gaz résiduel prélevé à partir du réacteur secondaire, ou dans lequel la synthèse de mélamine est réalisée dans une section de synthèse incluant un réacteur unique, et le gaz résiduel soumis audit procédé de purification est prélevé à partir dudit réacteur unique.

15. Procédé selon l'une quelconque des revendications précédentes, dans lequel le gaz résiduel (3) est introduit via un distributeur de gaz résiduel au-dessus ou au-dessous d'un niveau de liquide de la masse fondue d'urée collectée au bas de l'étage de purification.

16. Procédé combiné pour la synthèse d'urée et de mélamine dans lequel :
de l'ammoniac et du dioxyde de carbone sont mis en réaction pour former une solution d'urée dans une section de synthèse d'urée ; ladite solution est traitée dans au moins une section de récupération pour obtenir une solution d'urée purifiée ; l'eau est retirée de ladite solution pour former une masse fondue d'urée (14) ;
ladite masse fondue d'urée (14) est utilisée dans un procédé pour la synthèse de mélamine selon l'une quelconque des revendications 1 à 15 ;
des gaz résiduels (3) de mélamine générés pendant la synthèse de mélamine sont recyclés vers la production d'urée.
